# EUROPEAN PATENT SPECIFICATION

(11) **EP 3 190 978 B1**
(45) Date of publication and mention of the grant of the patent: **27.10.2021**
(21) Application number: 15763003.9
(22) Date of filing: 14.09.2015
(51) Int. Cl.: A61B 10/00, B01L 3/00, G01N 1/42, G01N 1/28

(54) **BIOLOGICAL SAMPLING AND STORAGE CONTAINER**
BEHÄLTER ZUR ENTNAHME UND LAGERUNG BIOLOGISCHER PROBEN
RÉCIPIENT D'ÉCHANTILLONNAGE ET DE STOCKAGE BIOLOGIQUE

(30) Priority: 12.09.2014 EP 14184535
(43) Date of publication of application: 19.07.2017
(73) Proprietor: VIB VZW, 9052 Gent (BE); Vrije Universiteit Brussel, 1050 Brussel (BE); Katholieke Universiteit Leuven K.U. Leuven R&D, 3000 Leuven (BE)
(72) Inventor: RAES, Jeroen, 2600 Berchem (BE); VANDEPUTTE, Doris, 3500 Hasselt (BE); JOOSSENS, Marie, 9000 Gent (BE); VAN LEEUWEN, Rianne, 2411 RW Bodegraven (NL)
(86) International application number: PCT/EP2015/070977
(87) International publication number: WO 2016/038224

(56) References cited:
- WO-A1-03/020426
- WO-A1-2013/192606
- US-A1- 2004 005 246
- US-A1- 2009 104 692
- US-A1- 2012 083 028
- US-A1- 2013 052 730
- None

## Description

### FIELD OF THE INVENTION

The invention relates to a biological sampling and storage container which allows easy sampling and aliquoting of a biological sample, as well as facilitated handling and storage of the samples and aliquots.

### BACKGROUND OF THE INVENTION

Access to frozen biological samples is of tremendous importance to improve the quality of certain analyses of biological samples. In order to be able to perform contemporary molecular biology technologies, such as microbial community analysis, metabolite analysis, RNA or DNA testing or protein (kinase, protease, nucleic hormone receptor...) activity testing, (freshly) frozen biological samples are often a prerequisite.

The problem is that biological samples taken at home (e.g. stool, urine or saliva samples from a patient) or during an expedition (e.g. soil samples) cannot be frozen immediately, and when frozen for instance in the freezer at the patients home or at the hospital, the sample still needs to be processed in a frozen state at the laboratory where the actual analysis of the sample will take place.

Currently, frozen clinical patient samples such as stool samples are not regarded as a routine source for diagnostic testing in clinical practice, and the routine practice does not include a simple and reliable solution for collecting, storing and preserving these samples.

However, when a disease such as cancer, a cardiovascular, an inflammatory or an infectious disease is suspected, the best hope for long-term survival for the patient is an accurate diagnosis in the earliest detectable stage of the disease. For this purpose various types of analysis of the sample need to occur. Some of these techniques might require a fresh or freshly frozen sample in order to preserve the sample from degradation, and to maintain e.g. the structure of the biological constituents in the sample. This is particularly necessary when performing contemporary molecular biology technologies such as e.g. microbial community analysis or metabolite analysis.

In case biological samples are frozen at an early stage, another issue is the processing of the sample at the laboratory. For certain analyses of the biological samples specific amounts of the sample need to be separated, something which is difficult when dealing with a frozen sample. The division of the frozen biological sample into smaller parts or aliquots for storage and further analysis is difficult as the sample needs to remain in a frozen state. The frozen condition of the sample provides many difficulties for aliquoting. For blood and stool samples, drilling devices such as the Cryoxtract are available. However, due to the variability of stool samples (amount, consistency, structure), these drilling devices only offer a solution for a very limited amount of stool samples.

A problem encountered with the sampling itself, in particular with faecal sampling, is that people are generally very reluctant of collecting faecal samples, mainly for hygienic reasons. Indeed, current methods for collecting faeces are generally people-unfriendly requiring the patient or staff to collect the stool and transfer it into a plastic jar, which is often too small or difficult to hygienically transfer the stool. Apart from the risk of potential contamination with urine or toilet water, and health risks, these methods not always provide reproducible or reliable results. The Fecotainer® has partially solved the above stated problems. It is a disposable collector that is simply placed under the toilet seat before use and can be well sealed with a stopper and lid after use, and then sent hygienically to a laboratory for further analysis. At the laboratory however, frozen samples still need to be aliquoted for certain molecular analyses.

Accordingly, there is a need for a system and method for acquiring, aliquoting, freezing and storing fresh biological samples such as stool samples, preferably at the location where the sample is taken, and in which the molecular activity is being preserved, and, which features a means for storing the frozen samples, allowing further examination and archiving at a laboratory or at a location different from the location where the sample is taken. WO 2013/192606 A1 discloses a biological sampling container according to the preamble of claim 1.

### SUMMARY OF THE INVENTION

The present invention provides a biological sampling and storage container as defined in claim 1.

In some embodiments, the aliquoting chambers (73) further comprise one or more vent openings (74).

In some embodiments, the aliquoting chambers (73) have a square, rectangular, rounded rectangular, oval rectangular, rectelliptical, squircular, oval or circular circumference.

In some embodiments, the aliquoting chambers (73) have a recessed and preferably a concave shape.

In some embodiments, the circumference of said aliquoting chambers (73) is wedge shaped.

In some embodiments, the top cover (3) is removably mounted onto the proximal end of said aliquoting element (7), the distal end of said aliquoting element (7) is removably mounted onto the proximal end of said sampling container (8) and said bottom cover (5) is removably mounted onto the distal end of said sampling container (8).

In some embodiments, interlocking features (11,12) which are not easily accessible to the user lock said top cover (3) to said aliquoting element (7) and said bottom cover (5) to said sampling container (8), and an interlocking feature (10) accessible to the user locks said aliquoting element (7) to said sampling container (8).

In some embodiments, the interlocking feature (10) accessible to the user is an external interlocking feature, preferably an external interlocking feature (10) comprising an external unlocking mechanism and/or the interlocking features (11,12) which are not easily accessible to the user are internal interlocking features, preferably internal interlocking features (11, 12) comprising a key triggered unlocking mechanism.

In some embodiments, the sampling container (8) comprises a sampling chamber (81) having upstanding walls parallel to the upstanding walls of said sampling container, wherein said upstanding walls of said sampling chamber (81) are smaller in height compared to said upstanding walls of said sampling container (8).

In some embodiments, the sampling chamber (81) comprises one or more sampling openings (82) corresponding to said aliquoting chambers (73) and the bottom cover (5) comprises extrusions (51) fitting into said sampling openings (82).

In some embodiments, the biological sampling and storage container (1) is made from a single type of material, preferably a temperature resistant material. In other embodiments, the biological sampling and storage container (1) is made from at least two different types of materials, preferably at least two different types of temperature resistant material.

In some embodiments, the biological sampling and storage container (1) additionally comprises one or more elements chosen from printed instructions, identification tags, sensing devices, temperature controlling means and/or tracing elements.

Also disclosed is a method for sampling and storing biological samples in the biological sampling and storage container according to the invention, comprising the steps of:
(a) opening said biological sampling and storage container by at least partially detaching said aliquoting element from said sampling container;
(b) inserting a biological sample in the sampling chamber of said sampling container;
(c) attaching the aliquoting element to said sampling container;
(d) optionally transporting said biological sampling and storage container;
(e) detaching the top and bottom cover from said sampling element;
(f) optionally detaching said sampling container from said aliquoting element;
(g) optionally, pressing the ejector, thereby ejecting the aliquots onto the top or bottom cover; and;
(h) optionally attaching the top cover to the bottom cover and storing the aliquots contained therein.

In some examples, the biological sampling and storage container is frozen prior to step (f). Yet another aspect of the invention relates to the use of the biological sampling and storage container according to the invention for the cryopreservation of biological samples.

### BRIEF DESCRIPTION OF THE DRAWINGS

Embodiments of the present invention will now be described, by way of example only, with reference to the accompanying drawings, in which:
**FIG. 1a** illustrates a bird's-eye view of the biological sampling and storage container assembly according to one of the embodiments of the present invention.
**FIG. 1b** illustrates a bird's-eye view of the storage container assembly according to one of the embodiments of the present invention.
**FIG. 2a** depicts a bird's-eye view illustration of the biological sampling and storage container according to one of the embodiments of the present invention.
**FIG. 2b** depicts a bottom-up view illustration of the biological sampling and storage container according to one of the embodiments of the present invention.
**FIG. 3a** illustrates a side view of the elements of the biological sampling and storage container according to one of the embodiments of the present invention.
**FIG. 3b** illustrates a side view of the biological sampling and storage container assembly according to one of the embodiments of the present invention.
**FIG. 3c** illustrates a side view of the storage container assembly according to one of the embodiments of the present invention.
**FIG. 4** illustrates a bird's-eye view of the stool collector according to one of the embodiments of the present invention.

### DETAILED DESCRIPTION OF THE INVENTION

The present invention aims to provide biological sampling and storage containers which solve one or more of the aforementioned disadvantages. Preferred embodiments of the present invention aim to provide biological sampling and storage containers which solve one or more of the aforementioned disadvantages. The present disclosure also aims to provide methods for collecting, aliquoting, transporting and storing biological samples, and in particular fecal samples, thereby solving one or more of the aforementioned disadvantages. Preferred embodiments of the present invention aim to provide methods for collecting, aliquoting, transporting and storing biological samples, and in particular fecal samples, thereby solving one or more of the aforementioned disadvantages.

To solve one or more of the above-described problems, at least one embodiment of the present invention adopts the following constructions as illustrated in the embodiments described below, some of which are also illustrated by the drawings. However, parenthesized or emboldened reference numerals affixed to respective elements merely exemplify the elements by way of example, with which it is not intended to limit the respective elements.

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the following detailed description of the invention, reference is made to the accompanying drawings that form a part hereof, and in which are shown by way of illustration only of specific embodiments in which the invention may be practiced. It is to be understood that other embodiments may be utilized and structural or logical changes may be made without departing from the scope of the present invention. The following detailed description, therefore, is not to be taken in a limiting sense.

The present invention provides in a biological sampling and storage container which allows easy sampling and aliquoting of the biological sample, fast and easy transport of the container, facilitated handling of the samples and aliquots by the lab technicians as well as simple and straightforward storage of the aliquots. In particular, the biological sampling and storage container allows an early freezing of the sample in the container shortly after the biological sample is taken, and the samples are aliquoted prior to freezing, without resulting in difficulties in handling the (frozen) samples and aliquots by the technicians which eventually manipulate the (frozen) samples for further analysis. The present invention further provides a user-friendly way of collecting a biological sample, in particular a fecal sample. There is minimal contact with the fecal material for the user, the container has a clean appearance and does not smell. In a first aspect, the present invention provides in a biological sampling and storage container comprising a top cover and a bottom cover adapted to fit onto each other; and a sampling element having a proximal end onto which the top cover fits and a distal end onto which the bottom cover fits, said sampling element further comprising:
- a sampling container comprising a sampling chamber for accepting the biological sample;
- an aliquoting element for dividing said biological sample into smaller parts, removably mounted on and in said sampling container, said
   aliquoting element comprising a structural element onto which one or more aliquoting chambers are positioned; and
- optionally, an ejector removably mounted onto said aliquoting element, wherein the ejector comprises one or more ejector wands fitting into said aliquoting chambers.

In a particular embodiment, the present invention provides in a biological sampling and storage container comprising a top cover and a bottom cover adapted to fit onto each other; and a sampling element having a proximal end onto which the top cover fits and a distal end onto which the bottom cover fits, said sampling element further comprising:
- a sampling container comprising a sampling chamber for accepting the biological sample;
- an aliquoting element for dividing said biological sample into smaller parts, removably mounted on and in said sampling container, said
   aliquoting element comprising a structural element onto which one or more aliquoting chambers are positioned, said aliquoting chambers comprising at least one ejector opening; and;
- an ejector removably mounted onto said aliquoting element, wherein the ejector comprises one or more ejector wands fitting into each ejector opening of said one or more aliquoting chambers.

As used in the present invention, the term "top cover" refers to the top part or lid of the biological sampling and storage container as well as the top part or lid of the biological sample storage container. As used in the present invention, the term "bottom cover" refers to the bottom part or base of the biological sampling and storage container as well as the bottom part or base of the biological sample storage container.

A biological sample storage container as defined herein is a container for storing a biological sample and comprises the top cover removably mounted onto the bottom cover. Preferably, the biological sample storage container as defined herein does not comprise a sampling element as defined herein. Preferably, both the top and bottom cover comprise upstanding walls coaxially fitting along each other. The height of the biological sample storage container ranges between about 5 mm and 30 mm, preferably between about 5 mm and 20 mm, more preferably between about 5 mm and 15 mm, such as for instance about 5 mm, about 7.5 mm, about 10 mm, about 12.5 mm or about 15 mm, allowing easy storage in freezers, preferably cryogenic freezers.

A biological sampling and storage container as taught herein is a container for biological sampling and storing of said biological sample. With the expression "biological sampling" is meant herein collecting or gathering of a biological sample.

To form the biological sampling and storage container, the top cover is mounted onto the proximal end of the sampling element and the bottom cover is mounted onto the distal end of the sampling element. Preferably, the top cover and the bottom cover are removably mounted onto the sampling element. This advantageously allows demounting the biological sampling and storage container in order to remove the sampling element, thereby leaving a biological sample storing container as taught herein. The height of the biological sampling and storage container ranges between about 10 mm and 50 mm, preferably between about 15 mm and 45 mm, more preferably between about 20 mm and 40 mm, such as for instance about 20 mm, about 25 mm, about 30 mm, about 35 mm or about 40 mm, allowing easy storage in freezers as well as transport.

As used in the present invention, the term "sampling element" refers to the part of the biological sampling and storage container into which the biological sample is introduced immediately after obtaining the sample. Apart from the sampling function, the sampling element comprises features which are used to aliquot the sample into smaller samples with a predefined size, shape and/or mass (also referred to as aliquoting). Preferably, the sampling element comprises three elements to allow the easy sampling and aliquoting of the biological sample. The bottom part of the sampling element is formed by a sampling container comprising a sampling chamber for accepting the biological sample. An aliquoting element is removably mounted on and in said sampling container. The aliquoting element comprising a structural element onto which one or more aliquoting chambers are positioned. The aliquoting chambers may comprise at least one ejector opening. Finally, an ejector is removably mounted onto said aliquoting element, wherein the ejector comprises an ejector wands fitting into the ejector openings of the aliquoting chamber. Depending on the size, shape and type of material of the aliquoting chamber, the ejector opening may not be required (as described further herein). As used in the present invention, the term "aliquot" refers to a part of the total sample, preferably an aliquot has a predefined shape, size and/or mass, resulting from the sample being pressed into the aliquoting chamber. The term "aliquoting" refer to the action of (sub)dividing or partitioning the sample into smaller parts (also referred to as aliquots) with a predefined shape, size and/or mass.

After introducing the biological sample into the sampling container, in particular into the sampling chamber, the aliquoting element is positioned on top of the sampling container and by exerting force, to mount the aliquoting element onto the sampling container, the sample is aliquoted. When exerting force to mount these two elements, the aliquoting chambers are pressed into the sample in the sampling chamber until the rims of the aliquoting chambers reach the bottom part of the sampling container, creating aliquots of the sample according to the size and shape of the aliquoting chambers. The aliquoting chambers are preferably provided with one or more vent openings, which allow air to remove from the aliquoting chamber upon aliquoting the sample. As a result the formed aliquots have the size, shape and/or mass predetermined by the aliquoting chamber, without trapped air remaining in the chamber and altering the size, shape and/or mass of the aliquots. At this moment in the sampling process the sample is already aliquoted and may be frozen. To remove the frozen aliquots from the aliquoting chambers, the ejector is preferably used. By exerting force onto the ejector, the ejector wands press the (frozen) aliquots out of the aliquoting chambers resulting in (frozen) aliquots with a predefined shape, size and/or mass ready for further (cryogenic) storage and/or biological analysis.

As used in the present invention, the term "biological sample" refers to a specimen or sample comprising biological material (i.e. material derived from living or lived organisms, including microorganisms, plants and animals). The sample or specimen may be obtained from a biological source such as animal or plant material, or other sources containing biological material such as soil. The biological sample may be obtained from an organism (patient) such as human or from components (e.g. tissue or cells) of such an organism. These biological samples may include stool or faeces samples, urine samples, saliva samples, biopsy samples, blood samples, or samples from other biological fluids. Preferably, said biological samples are faecal samples. Also envisaged herein as biological sample are for example, but without limitation, plant material (e.g., the peal of fruits/legumes, fermented cocoa fruits), sand samples, silt samples, clay samples, loam samples, sludge samples, wastewater samples, fermentation fluids, food ingredients (e.g., dough for bread, sugarcane processing, gluten extract out of wheat flour), food products, waste products of industrial processes (e.g. yeast and protein matrix of a fermentation process).

Biological samples sampled, aliquoted, frozen, and/or eventually stored in a container as taught herein are particularly suitable for analyses such as DNA or RNA testing, protein activity testing, metabolite analysis, microbial community analysis, microbial analysis, preferably for microbial analysis.

In particular for medical samples such as faecal samples, molecular biology techniques, such as e.g. microbial community analysis, metabolite analysis, DNA or RNA testing, or protein activity testing, require fast freezing of the sample after collection. This typically needs to occur already at the patients home, prior to the transport of the samples to the laboratory or hospital. Hygienic issues are of great importance as the samples will need to be frozen in the home freezer typically used for freezing food. The biological sampling and storage container according to the present invention provides in a hygienic and easy-to-use collection system for sampling stool samples. The patient from which the stool sample is taken only needs to open the container by demounting the aliquoting element from the sampling container and introduce the sample in the sampling container, in particular the sampling chamber. After closing the container (by mounting the aliquoting element onto the sampling container) the container can be easily handled and manipulated. After freezing, the container can be transported to the laboratory of hospital where the top cover and bottom cover are removed. The aliquots generated upon closing the container can be removed and stored into the biological sample storage container formed by the top and bottom cover. The container according to the present invention no longer requires defrosting the samples and it allows both the patients and the technicians handling the samples at the laboratory to work efficiently and in highly hygienic circumstances.

The sampling container according to the present invention comprises a sampling chamber into which the sample can be introduced. The sampling chamber is preferably smaller than the sampling container to allow space for the potential overflow of the sample. Upon pressing the aliquoting element into the sampling chamber the sample is aliquoted into smaller portions, but part of the sample is also pushed aside. The smaller sampling chamber provides overflow spaces in the sampling container allowing to accept the surplus of the sample which has not been aliquoted. Also, the entire sample remains in the container, together with the aliquots. Therefore, apart from storing and analyzing the aliquots in the laboratory, also the remaining parts of the sample (i.e. the not aliquoted part of the sample) are available for the technicians to perform additional tests, preferably tests which do not require samples with a predefined shape, size and/or mass. The not aliquoted part of the stool sample, but also the aliquoted part of the stool sample, may be used for a fecal occult blood test (to detect bleeding in the gastrointestinal system), the microscopic examination of the stool sample to detect for instance parasitic diseases such as ascariasis, hookworm, strongyloidiasis and whipworm, or other (medical) tests to detect bacterial diseases, toxins or viruses.

The aliquoting element is the part of the sampling container that forms the aliquots of the sample. The aliquoting element comprises a structural element which allows the mounting of the aliquoting element onto the sampling container and also preferably allows mounting the top cover onto the aliquoting element. The aliquoting element further comprises one or more aliquoting chambers extending from the structural element. The aliquoting chambers are half open shaped recessed structures preferably positioned at the end of an extension structure and extending onto the bottom wall of the sampling chamber. As a result, the bottom wall of the sampling chamber together with the recessed aliquoting chamber forms the shape, size and/or mass of the aliquot. Each of the aliquoting chambers may comprise at least one ejector opening into which an ejector wand fits. After freezing the aliquots, pressing the ejector will result in the ejector wands to protrude onto the aliquoting chambers, thereby removing the aliquots. Depending on the size, shape and type of material of the recessed aliquoting chamber, an ejector opening is not required. This may for example be the case if a more elastic material (for example, synthetic rubber amongst other) is used for the aliquoting chamber. In such a case, pressing the ejector will result in the ejector wands to protrude onto the aliquoting chamber, thereby deforming the elastic material of the aliquoting chamber and removing the aliquots.

The ejector is the part of the sampling element that is used to eject the aliquots from the aliquoting chambers. The ejector comprises one or more ejector wands each extending coaxially through the extension structure of the aliquoting element and ending onto an aliquoting chamber or in the ejector opening of an aliquoting chamber, as the case may be.

It will be understood by a skilled person that the number of aliquoting chambers in the biological sampling and storage container depends on the type of sample that needs to be taken and the amount of experimental tests that needs to occur. Therefore, the biological sampling and storage container according to the present invention may comprise between 1 and 100 aliquoting chambers, or even more. Preferably said biological sampling and storage container comprises 1, 2, 4, 6, 8, 10, 12, 16, 20, 24, 36 or 96 aliquoting chambers.

Preferably the present invention provides in a biological sampling and storage container as described herein, wherein said aliquoting chambers further comprise one or more vent openings. Preferably the one or more vent openings are positioned at the top of the aliquoting chamber (i.e. adjacent to the ejector opening), more preferably in the side wall of the aliquoting chamber adjacent to the ejector opening. Said top of the aliquoting chamber contacts the sample introduced in the sample chamber last. By providing one or more vent openings at the top of the aliquoting chambers, air is allowed to be removed from the top of the aliquoting chamber upon aliquoting the sample. As a result the formed aliquots have the size, shape and/or mass predetermined by the aliquoting chamber, without trapped air remaining in the chamber and altering the size, shape and/or mass of the aliquots.

According to a preferred embodiment, the biological sampling and storage container according to the present invention provides that said aliquoting chambers have a square, rectangular, rounded rectangular, oval rectangular, rectelliptical, squircular, oval or circular circumference. The circumference or edge of the aliquoting chambers refers to the shape of the rim of the aliquoting chamber that makes contact with the bottom wall of the sampling chamber. Preferably, said shape is rounded rectangular, oval rectangular, oval or circular. In a preferred embodiment, said aliquoting chambers have a recessed and preferably a concave shape. The shapes of the aliquoting chamber are such that aliquots are provided having particular shapes, sizes and/or masses conform to the requirements of the analysis techniques in which the aliquots are used. This facilitates the handling of the aliquots, maintains a high hygienic standard and provides a high degree of standardization.

Preferably the present invention provides in a biological sampling and storage container as described herein, wherein the circumference of said aliquoting chambers is wedge shaped. By providing that the rims of the aliquoting chambers are wedge or V-shaped, the aliquoting procedure may be improved, particularly for harder samples. The wedge or V-shaped rim of the aliquoting chamber acts as a knife, cutting the sample and forming the aliquots without requiring the need to exert an excessive amount of force for mounting the aliquoting element onto the sampling container. In an embodiment the rims of said aliquoting chambers are at least partially made from a metallic material.

In a preferred embodiment, the biological sampling and storage container as described herein provides that the top cover is removably mounted onto the proximal end of the aliquoting element, that the distal end of said aliquoting element is removably mounted onto the proximal end of the sampling container and that the bottom cover is removably mounted onto the distal end of the sampling container. By mounting the top cover onto the proximal end of the aliquoting element, the ejector is protected from accidental impulses which may trigger the ejector thereby ejecting the aliquots in an accidental manner.

More preferably, the biological sampling and storage container as described herein provides that an interlocking feature which is not easily accessible to the user locks said top cover to said aliquoting element and said bottom cover to said sampling container, and wherein an interlocking feature accessible to the user locks said aliquoting element to said sampling container. It is clear from the use of the container as disclosed herein that upon sampling, the aliquoting element needs at least partially to be demounted from the sampling container, but it is only when the samples are aliquoted and need to be stored that a technician will demount the top and bottom cover to eject the aliquots for further storage. Using an interlocking feature which is not easily accessible for the user for locking the top cover to the aliquoting element and the bottom cover to the sampling container, provides that the top and bottom cover will not be removed accidentally upon sampling the samples. Only the interlocking feature locking the aliquoting element to the sampling container is accessible to the user taking the sample thereby reducing the risk of accidentally opening parts of the container that are intended to be opened later. An interlocking feature which is not easily accessible to the user may be an internal interlocking feature, an interlocking feature comprising a key triggered unlocking mechanism, or an internal interlocking feature comprising a key triggered unlocking mechanism. An interlocking feature accessible to the user may be an external interlocking feature such as an external unlocking feature with an external unlocking mechanism. The interlocking mechanism may be a snap fit locking mechanism, e.g. a snap fit locking mechanism with manual triggered unlocking mechanism, which is accessible to the user, or a snap fit lock mechanism with key triggered unlocking mechanism, which is not easily acceptable to the user.

The biological sampling and storage container as described herein provides that said sampling container is configured to receive a sampling chamber. In an embodiment, the biological sampling and storage container as described herein provides that said sampling container comprises a sampling chamber having upstanding walls parallel to the upstanding walls of said sampling container, wherein said upstanding walls of said sampling chamber are smaller in height compared to said upstanding walls of said sampling container. In order to create an overflow region in the sampling container for non-aliquoted sample, the height of the sampling chamber is preferably smaller compared to the height of the sampling container, preferably said sampling chamber is 10%, 20%, 30%, 40% or 50% smaller in height compared to the height of the sampling container.

In a preferred embodiment, the biological sampling and storage container as described herein provides that said sampling chamber comprises one or more sampling openings corresponding to said aliquoting chambers and wherein said bottom cover comprises extrusions fitting into said sampling openings. By providing sampling openings in the bottom of the sampling chamber corresponding to the aliquoting chambers and into which the extrusions in the bottom cover fit the removal of the aliquots is facilitated. By removing the bottom cover from the biological sampling and storage container the bottom part of the aliquots becomes reachable and upon ejecting the aliquots these can be intercepted without having to demount the aliquoting element from the sampling container. As a result, in the laboratory, the non-aliquoted sample is not exposed and the aliquots can be ejected separately into the biological sample storage container.

In embodiments, the biological sampling and storage container as taught herein is made from a material having a glass transition temperature of -80°C or lower, in order to allow storage of the container in freezers, in particular cryogenic freezers.

In a preferred embodiment, the biological sampling and storage container as described herein provides that said biological sampling and storage container is made from a single type of material, preferably temperature resistant material. Alternatively, the biological sampling and storage container as described herein provides that said biological sampling and storage container is made from a combination of at least two different types of material, preferably at least two types of temperature resistant material. The choice of a suitable material may depend on the type and resolution of the biological sample, the purpose of the sampling, etc. Preferred types of material include materials having a heat conductivity of at least 0.1 W/mK, more particularly, at least 0.2, 0.3, 0.4, 0.5, 0.75 or 1 W/mK. For example, the material used for the specimen container may be a (synthetic) polymer such as plastic (such as polypropylene, or polyethylene), more particularly a reinforced plastic or a cryoplastic, or a metallic material. A reinforced plastic material refers to a composite material made of a polymer matrix reinforced with fibers. The fibers are usually glass, carbon, or aramid. In particular embodiments, the container as taught herein is made from a (synthetic) polymer. The polymer may be an epoxy, vinylester or polyester thermosetting plastic, or high density polyethylene (HDPE), preferably HDPE. The polymer may also be low density polyethylene (LDPE), preferably LDPE. Other examples of suitable material include synthetic rubber, such as styrene ethylene butylene styrene (SEBS), or thermoplastic elastomers (TPE), such as styrenic block copolymers (TPE-s), polyolefin blends (TPE-o), elastomeric alloys (TPE-v or TPV), thermoplastic polyurethanes (TPU), thermoplastic copolyester.

In a preferred embodiment, the biological sampling and storage container as described herein additionally comprises one or more elements chosen from printed instructions, identification tags, sensing devices, temperature controlling means and tracing elements. Identification tags are devices enabling the identification and traceability of the sample such as a barcode, data matrix or similar structures. Sensing devices are tools used to monitor environmental parameters such as for instance a temperature monitoring sensor, a time monitoring sensors or sensors monitoring other environmental parameters such as humidity. Temperature controlling means are features which are used to control the temperature stability of the container as described herein, including for instance cooling elements, heating elements, isolation elements or similar elements. Tracing elements are features which allow the identification of the position of the container, or the position where the sample was taken, including for instance GPS devices.

Also disclosed is a method for sampling and storing biological samples in the biological sampling and storage container according to the present invention and as described herein, comprising the steps of:
(a) opening said biological sampling and storage container by at least partially detaching said aliquoting element from said sampling container;
(b) inserting a biological sample in the sampling chamber of said sampling container;
(c) attaching the aliquoting element to said sampling container;
(d) optionally transporting said biological sampling and storage container;
(e) detaching the top and bottom cover from said sampling element;
(f) optionally detaching said sampling container from said aliquoting element;
(g) pressing the ejector, thereby ejecting the aliquots onto the top or bottom cover; and;
(h) optionally attaching the top cover to the bottom cover and storing the aliquots contained therein.

Preferably, said biological sampling and storage container is frozen prior to step (f). For example, said freezing step may take place after step (c), or after optional step (d), or after step (e).

In a further aspect, the present invention provides in the use of the biological sampling and storage container according to the present invention for the cryopreservation of biological samples.

Also disclosed is a kit for sampling and storing biological samples in the biological sampling and storage container, comprising the biological sampling and storage container according to the present invention, a spoon for introducing the sample in the container, a set of instructions on how to use the biological sampling and storage container and optionally other sampling means such as for instance stool collection means.

### EXAMPLES

The present example provides a particular embodiment according to the invention.

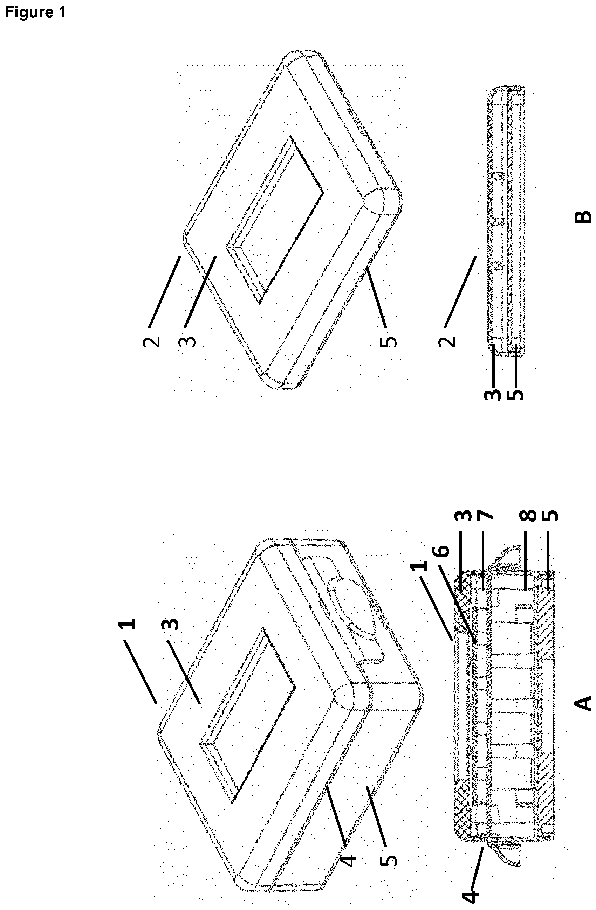

Figure 1 illustrates the biological sampling and storage container according to a particular embodiment of the present invention, which, depending on the assembled parts may form the sampling container assembly (1) as represented in figure 1A or the storage container assembly (2) as represented in figure 1B. The sampling container assembly (1) comprises a top cover (3) fitted onto the proximal end of the sampling element (4) and a bottom cover (5) fitting onto the distal end of the sampling element (4). The sampling element (4) comprises an ejector (6), an aliquoting element (7) and a sampling container (8). The storage container assembly (2) comprises a top cover (3) fitted onto the bottom cover (5).

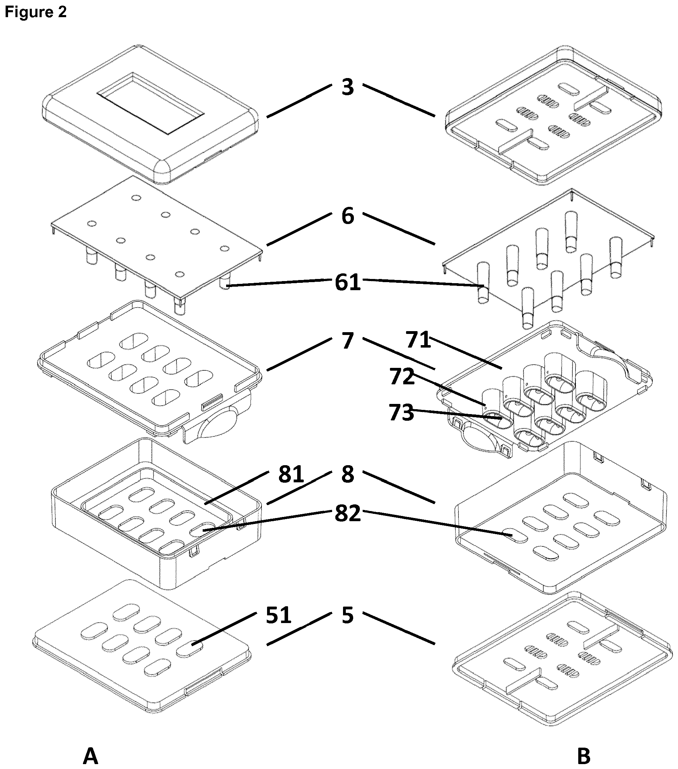

Figure 2 illustrates more details of the biological sampling and storage container according to a particular embodiment of the present invention. The sampling element (4) comprises of an ejector (6) comprising ejector wands (61) (eight in the depicted embodiment); an aliquoting element (7) comprising a structural element (71) from which the aliquoting chambers (73) extend through an extension structure (72); and a sampling container (8) comprising a sampling chamber (81) for accepting the biological sample. The depicted aliquoting chambers (73) have a concave shape and an oval rectangular circumference.

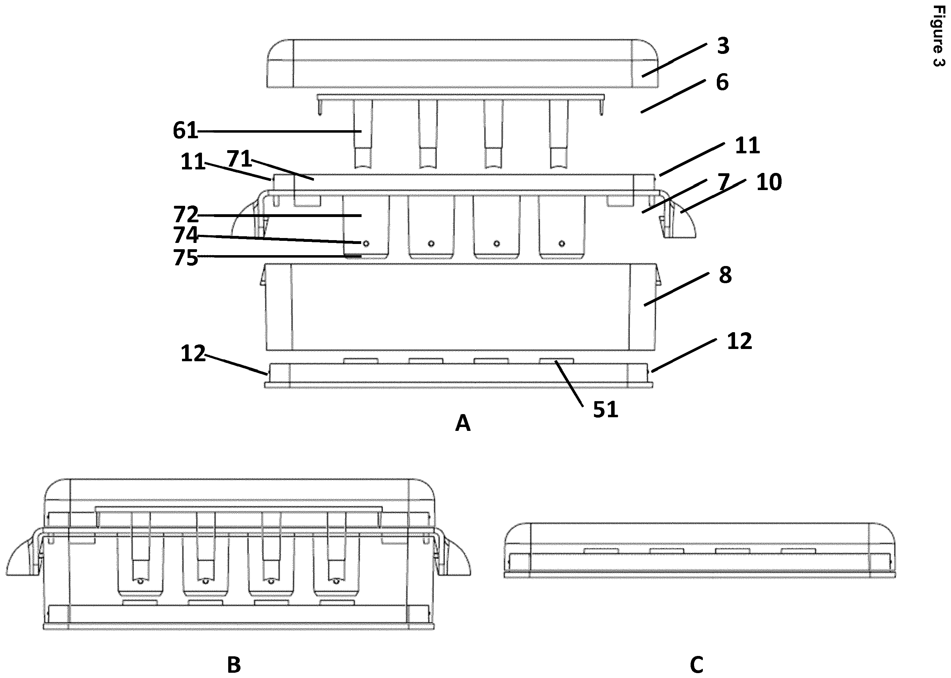

The depicted biological sampling and storage container further provides that the sampling chamber (81) comprises sampling openings (82) corresponding to the aliquoting chambers (73). The bottom cover (5) comprises extrusions (51) fitting into the sampling openings (82). Figure 3 shows a side view of the biological sampling and storage container according to a particular embodiment of the present invention. In figure 3 the vent openings (74) at the top of the aliquoting chamber (73) and the wedge shaped circumference (75) of the aliquoting chambers (73) are shown. Also, an internal interlocking feature (11) locking the top cover (3) to the aliquoting element (7) and an internal interlocking feature (12) locking the bottom cover (5) to the sampling container (8) are shown. The aliquoting element (7) is locked to the sampling container (8) with an external interlocking feature (10) comprising an external unlocking mechanism.

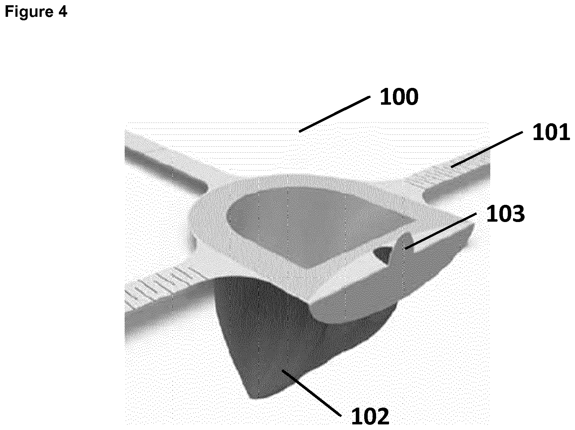

Figure 4 illustrates a stool collector (100) which may be used in association with the biological sampling and storage container according to one of the embodiments of the present invention. For collecting stool samples in a hygienic manner, a stool collector (100) as depicted is attached to the toilet seat using attachment means (101). The attachment means (101) hold the collection bag (102) in the center of the toilet. The stool collector (100) further comprises a position indicator (103) which allows an indication of the correct position for collecting the feces samples without cross contamination with urine.

## Claims

1. A biological sampling and storage container (1) comprising a top cover (3) and a bottom cover (5) adapted to fit onto each other; and a sampling element (4) having a proximal end onto which the top cover (3) fits and a distal end onto which the bottom cover (5) fits, wherein said sampling element (4) further comprises:
a. a sampling container (8) comprising a sampling chamber (81) for accepting the biological sample and **characterized in that** the sampling element further comprises
b. an aliquoting element (7) for dividing said biological sample into smaller parts, removably mounted on and in said sampling container, said aliquoting element comprising a structural element (71) onto which one or more aliquoting chambers (73) are positioned.

2. The biological sampling and storage container (1) according to claim 1, further comprising an ejector (6) removably mounted onto said aliquoting element, wherein the ejector comprises ejector wands (61) fitting into said aliquoting chambers.

3. The biological sampling and storage container (1) according to claim 2, wherein said aliquoting chambers (73) comprise at least one ejector opening and wherein said ejector comprises ejector wands (61) fitting into each ejector opening of said aliquoting chambers.

4. The biological sampling and storage container (1) according to any of claims 1 to 3, wherein said aliquoting chambers (73) further comprise one or more vent openings (74).

5. The biological sampling and storage container (1) according to any of claims 1 to 4, wherein said aliquoting chambers (73) have a square, rectangular, rounded rectangular, oval rectangular, rectelliptical, squircular, oval or circular circumference.

6. The biological sampling and storage container (1) according to any of claims 1 to 5, wherein said aliquoting chambers (73) have a recessed and preferably a concave shape.

7. The biological sampling and storage container (1) according to any of claims 1 to 6, wherein the circumference of said aliquoting chambers (73) is wedge shaped.

8. The biological sampling and storage container (1) according to any of claims 1 to 7, wherein said top cover (3) is removably mounted onto the proximal end of said aliquoting element (7), wherein the distal end of said aliquoting element (7) is removably mounted onto the proximal end of said sampling container (8) and wherein said bottom cover (5) is removably mounted onto the distal end of said sampling container (8).

9. The biological sampling and storage container (1) according to any of claims 1 to 8, wherein interlocking features (11, 12) which are not easily accessible to the user lock said top cover (3) to said aliquoting element (7) and said bottom cover (5) to said sampling container (8), and wherein an interlocking feature (10) accessible to the user locks said aliquoting element (7) to said sampling container (8).

10. The biological sampling and storage container (1) according to claim 9, wherein said interlocking feature (10) accessible to the user is an external interlocking feature, preferably an external interlocking feature (10) comprising an external unlocking mechanism and/or wherein said interlocking features (11, 12) which are not easily accessible to the user are internal interlocking features, preferably internal interlocking features (11, 12) comprising a key triggered unlocking mechanism.

11. The biological sampling and storage container (1) according to any of claims 1 to 10, wherein said sampling container (8) comprises a sampling chamber (81) having upstanding walls parallel to the upstanding walls of said sampling container, wherein said upstanding walls of said sampling chamber (81) are smaller in height compared to said upstanding walls of said sampling container (8).

12. The biological sampling and storage container (1) according to any of claims 1 to 11, wherein said sampling chamber (81) comprises one or more sampling openings (82) corresponding to said aliquoting chambers (73) and wherein said bottom cover (5) comprises extrusions (51) fitting into said sampling openings (82).

13. The biological sampling and storage container (1) according to any of claims 1 to 12, wherein said biological sampling and storage container (1) is made from a single type of material, preferably a temperature resistant material, or from at least two different types of materials, preferably at least two different types of temperature resistant materials.

14. The biological sampling and storage container (1) according to any of claims 1 to 13, additionally comprising one or more elements chosen from printed instructions, identification tags, sensing devices, temperature controlling means and/or tracing elements.

15. Use of the biological sampling and storage container according to claims 1 to 14 for the cryopreservation of biological samples.

## Patentansprüche

1. Biologischer Probenahme- und Lagerbehälter (1), umfassend eine obere Abdeckung (3) und eine untere Abdeckung (5), die dazu eingerichtet sind, aufeinander zu passen; und ein Probenahmeelement (4) mit einem proximalen Ende, auf das die obere Abdeckung (3) passt, und einem distalen Ende, auf das die untere Abdeckung (5) passt, wobei das Probenahmeelement (4) weiterhin umfasst:
a. einen Probenahmebehälter (8), umfassend eine Probenahmekammer (81) zum Aufnehmen der biologischen Probe, und **dadurch gekennzeichnet, dass** das Probenahmeelement weiterhin umfasst:
b. ein Aliquotierungselement (7) zum Aufteilen der biologischen Probe in kleinere Teile, das abnehmbar auf und in dem Probenahmebehälter montiert ist, wobei das Aliquotierungselement ein Strukturelement (71) umfasst, auf dem eine oder mehrere Aliquotierungskammern (73) positioniert sind.

2. Biologischer Probenahme- und Lagerbehälter (1) nach Anspruch 1, weiterhin umfassend eine Ausstoßvorrichtung (6), die abnehmbar auf dem Aliquotierungselement montiert ist, wobei die Ausstoßvorrichtung Ausstoßvorrichtungswände (61) umfasst, die in die Aliquotierungskammern passen.

3. Biologischer Probenahme- und Lagerbehälter (1) nach Anspruch 1, wobei die Aliquotierungskammern (73) mindestens eine Ausstoßvorrichtungsöffnung umfassen und wobei die Ausstoßvorrichtung Ausstoßvorrichtungswände (61) umfasst, die in jede Ausstoßvorrichtungsöffnung der Aliquotierungskammern passen.

4. Biologischer Probenahme- und Lagerbehälter (1) nach einem der Ansprüche 1 bis 3, wobei die Aliquotierungskammern (73) weiterhin eine oder mehrere Lüftungsöffnungen (74) umfassen.

5. Biologischer Probenahme- und Lagerbehälter (1) nach einem der Ansprüche 1 bis 4, wobei die Aliquotierungskammern (73) einen quadratischen, rechteckigen, abgerundeten rechteckigen, ovalen rechteckigen, rechteckig-elliptischen, quadratisch-kreisförmigen, ovalen oder kreisförmigen Umfang aufweisen.

6. Biologischer Probenahme- und Lagerbehälter (1) nach einem der Ansprüche 1 bis 5, wobei die Aliquotierungskammern (73) eine vertiefte und vorzugsweise eine konkave Form aufweisen.

7. Biologischer Probenahme- und Lagerbehälter (1) nach einem der Ansprüche 1 bis 6, wobei der Umfang der Aliquotierungskammern (73) keilförmig ist.

8. Biologischer Probenahme- und Lagerbehälter (1) nach einem der Ansprüche 1 bis 7, wobei die obere Abdeckung (3) abnehmbar auf dem proximalen Ende des Aliquotierungselements (7) montiert ist, wobei das distale Ende des Aliquotierungselements (7) abnehmbar auf dem proximalen Ende des Probenahmebehälters (8) montiert ist und wobei die untere Abdeckung (5) abnehmbar auf dem distalen Ende des Probenahmebehälter (8) montiert ist.

9. Biologischer Probenahme- und Lagerbehälter (1) nach einem der Ansprüche 1 bis 8, wobei Verzahnungsmerkmale (11, 12), die für den Benutzer nicht leicht zugänglich sind, die obere Abdeckung (3) mit dem Aliquotierungselement (7) und die untere Abdeckung (5) mit dem Probenahmebehälter (8) verriegeln, und wobei ein Verzahnungsmerkmal (10), das für den Benutzer zugänglich ist, das Aliquotierungselement (7) mit dem Probenahmebehälter (8) verriegelt.

10. Biologischer Probenahme- und Lagerbehälter (1) nach Anspruch 9, wobei das Verzahnungsmerkmal (10), das für den Benutzer zugänglich ist, ein externes Verzahnungsmerkmal, vorzugsweise ein externes Verzahnungsmerkmal (10) ist, umfassend einen externen Entriegelungsmechanismus, und/oder wobei die Verzahnungsmerkmale (11, 12), die für den Benutzer nicht leicht zugänglich sind, interne Verzahnungsmerkmale, vorzugsweise interne Verzahnungsmerkmale (11, 12) sind, umfassend einen durch einen Schlüssel ausgelösten Entriegelungsmechanismus.

11. Biologischer Probenahme- und Lagerbehälter (1) nach einem der Ansprüche 1 bis 10, wobei der Probenahmebehälter (8) eine Probenahmekammer (81) mit aufrechten Wänden, die parallel zu den aufrechten Wänden des Probenahmebehälters sind, umfasst, wobei die aufrechten Wände der Probenahmekammer (81) eine kleinere Höhe verglichen mit den aufrechten Wänden des Probenahmebehälters (8) aufweisen.

12. Biologischer Probenahme- und Lagerbehälter (1) nach einem der Ansprüche 1 bis 11, wobei die Probenahmekammer (81) eine oder mehrere Probenahmeöffnungen (82) umfasst, die den Aliquotierungskammern (73) entsprechen, und wobei die untere Abdeckung (5) Extrusionen (51) umfasst, die in die Probenahmeöffnungen (82) passen.

13. Biologischer Probenahme- und Lagerbehälter (1) nach einem der Ansprüche 1 bis 12, wobei der biologische Probenahme- und Lagerbehälter (1) aus einem einzigen Materialtyp, vorzugsweise einem temperaturbeständigen Material, oder aus mindestens zwei unterschiedlichen Materialtypen, vorzugsweise mindestens zwei unterschiedlichen temperaturbeständigen Materialtypen, hergestellt ist.

14. Biologischer Probenahme- und Lagerbehälter (1) nach einem der Ansprüche 1 bis 13, zusätzlich umfassend ein oder mehrere Elemente, die aus gedruckten Anweisungen, Identifikationsetiketten, Erfassungsvorrichtungen, Temperaturregulierungsmitteln und/oder Tastelementen ausgewählt sind.

15. Verwendung des biologischen Probenahme- und Lagerbehälters nach den Ansprüchen 1 bis 14 zur Kryokonservierung von biologischen Proben.

## Revendications

1. Récipient d'échantillonnage et de stockage biologique (1) comprenant un couvercle supérieur (3) et un couvercle inférieur (5) adaptés à s'ajuster l'un sur l'autre ; et un élément d'échantillonnage (4) ayant une extrémité proximale sur laquelle le couvercle supérieur (3) s'ajuste et une extrémité distale sur laquelle le couvercle inférieur (5) s'ajuste, dans lequel ledit élément d'échantillonnage (4) comprend en outre :
a. un récipient d'échantillonnage (8) comprenant une chambre d'échantillonnage (81) pour accepter l'échantillon biologique et **caractérisé en ce que** l'élément d'échantillonnage comprend en outre
b. un élément d'aliquotage (7) pour diviser ledit échantillon biologique en parties plus petites, monté de manière amovible sur et dans ledit récipient d'échantillonnage, ledit élément d'aliquotage comprenant un élément structurel (71) sur lequel une ou plusieurs chambres d'aliquotage (73) sont positionnées.

2. Récipient d'échantillonnage et de stockage biologique (1) selon la revendication 1, comprenant en outre un éjecteur (6) monté de manière amovible sur ledit élément d'aliquotage, dans lequel l'éjecteur comprend des tiges d'éjecteur (61) s'ajustant dans lesdites chambres d'aliquotage.

3. Récipient d'échantillonnage et de stockage biologique (1) selon la revendication 2, dans lequel lesdites chambres d'aliquotage (73) comprennent au moins une ouverture d'éjecteur et dans lequel ledit éjecteur comprend des tiges d'éjecteur (61) s'ajustant dans chaque ouverture d'éjecteur desdites chambres d'aliquotage.

4. Récipient d'échantillonnage et de stockage biologique (1) selon l'une quelconque des revendications 1 à 3, dans lequel lesdites chambres d'aliquotage (73) comprennent en outre une ou plusieurs ouvertures de ventilation (74).

5. Récipient d'échantillonnage et de stockage biologique (1) selon l'une quelconque des revendications 1 à 4, dans lequel lesdites chambres d'aliquotage (73) ont une circonférence carrée, rectangulaire, rectangulaire arrondie, rectangulaire ovale, rectelliptique, squirculaire, ovale ou circulaire.

6. Récipient d'échantillonnage et de stockage biologique (1) selon l'une quelconque des revendications 1 à 5, dans lequel lesdites chambres d'aliquotage (73) ont une forme encastrée et de préférence concave.

7. Récipient d'échantillonnage et de stockage biologique (1) selon l'une quelconque des revendications 1 à 6, dans lequel la circonférence desdites chambres d'aliquotage (73) est en forme de coin.

8. Récipient d'échantillonnage et de stockage biologique (1) selon l'une quelconque des revendications 1 à 7, dans lequel ledit couvercle supérieur (3) est monté de manière amovible sur l'extrémité proximale dudit élément d'aliquotage (7), dans lequel l'extrémité distale dudit élément d'aliquotage (7) est montée de manière amovible sur l'extrémité proximale dudit récipient d'échantillonnage (8) et dans lequel ledit couvercle inférieur (5) est monté de manière amovible sur l'extrémité distale dudit récipient d'échantillonnage (8).

9. Récipient d'échantillonnage et de stockage biologique (1) selon l'une quelconque des revendications 1 à 8, dans lequel des fonctions de verrouillage (11, 12) qui ne sont pas aisément accessibles à l'utilisateur verrouillent ledit couvercle supérieur (3) audit élément d'aliquotage (7) et ledit couvercle inférieur (5) audit récipient d'échantillonnage (8), et dans lequel une fonction de verrouillage (10) accessible à l'utilisateur verrouille ledit élément d'aliquotage (7) audit récipient d'échantillonnage (8).

10. Récipient d'échantillonnage et de stockage biologique (1) selon la revendication 9, dans lequel ladite fonction de verrouillage (10) accessible à l'utilisateur est une fonction de verrouillage externe, de préférence une fonction de verrouillage externe (10) comprenant un mécanisme de déverrouillage externe et/ou dans lequel lesdites fonctions de verrouillage (11, 12) qui ne sont pas aisément accessibles à l'utilisateur sont des fonctions de verrouillage internes, de préférence des fonctions de verrouillage internes (11, 12) comprenant un mécanisme de déverrouillage déclenché par une clé.

11. Récipient d'échantillonnage et de stockage biologique (1) selon l'une quelconque des revendications 1 à 10, dans lequel ledit récipient d'échantillonnage (8) comprend une chambre d'échantillonnage (81) ayant des parois verticales parallèles aux parois verticales dudit récipient d'échantillonnage, dans lequel lesdites parois verticales de ladite chambre d'échantillonnage (81) sont plus petites en hauteur par rapport auxdites parois verticales dudit récipient d'échantillonnage (8).

12. Récipient d'échantillonnage et de stockage biologique (1) selon l'une quelconque des revendications 1 à 11, dans lequel ladite chambre d'échantillonnage (81) comprend une ou plusieurs ouvertures d'échantillonnage (82) correspondant auxdites chambres d'aliquotage (73) et dans lequel ledit couvercle inférieur (5) comprend des extrusions (51) s'ajustant dans lesdites ouvertures d'échantillonnage (82).

13. Récipient d'échantillonnage et de stockage biologique (1) selon l'une quelconque des revendications 1 à 12, dans lequel ledit récipient d'échantillonnage et de stockage biologique (1) est réalisé en un seul type de matériau, de préférence un matériau résistant à la température, ou en au moins deux types différents de matériaux, de préférence au moins deux types différents de matériaux résistant à la température.

14. Récipient d'échantillonnage et de stockage biologique (1) selon l'une quelconque des revendications 1 à 13, comprenant en outre un ou plusieurs éléments choisis parmi des instructions imprimées, étiquettes d'identification, dispositifs de détection, moyens de commande de température et/ou éléments de traçage.

15. Utilisation du récipient d'échantillonnage et de stockage biologique selon les revendications 1 à 14 pour la cryoconservation d'échantillons biologiques.
